# EUROPEAN PATENT APPLICATION

(11) **EP 2 883 520 A1**
(43) Date of publication of application: **17.06.2015**
(21) Application number: 13196605.3
(22) Date of filing: 11.12.2013
(51) Int. Cl.: A61F 5/44, A61F 5/449

(54) **A double needle bed warp-knitted urine bag fixation garment**

(71) Applicant: TYTEX A/S, 7430 Ikast (DK)
(72) Inventor: Rasmussen, Knud Fjelsted, 7430 Ikast (DK); Ærenlund, Jacob, 7430 Ikast (DK)
(74) Representative: Hoffmann Dragsted A/S

(57) **Abstract**

The present invention relates to a double needle bed warp-knitted urine bag fixation garment for fixating a urine bag to a leg of a wearer, comprising a tubular part having a first end and a second end and a first layer and a second layer extending between the first end and the second end, the tubular part having circumferential direction, the tubular part having an upper part and a lower part, the lower part extending from the upper part towards the second end, the first layer having a first rib edge at the first end, and the second layer having a second rib edge at the first end, the first layer and the second layer being interknitted along substantially the entire circumferential direction at the second end, and the second end having a third rib edge, so that the tubular part is substantially closed between the first layer and the second layer at the second end and open between the first layer and the second layer at the first end, thereby defining a room between the first layer and the second layer, the room being accessible from the first end and the room being adapted to accommodate the urine bag, wherein the tubular part comprises a connection in which the first layer and the second layer are connected along a part of the circumferential direction of the tubular part at a predetermined distance from the first end for providing a pocket for the urine bag in the room, the connection being adapted to maintain the urine bag in position in the pocket while it is filled during use.

## Description

### Field of the invention

The present invention relates to a double needle bed warp-knitted urine bag fixation garment for fixating a urine bag to a leg of a wearer, comprising a tubular part having a first end and a second end and a first layer and a second layer extending between the first end and the second end, the tubular part having circumferential direction, the tubular part having an upper part and a lower part, the lower part extending from the upper part towards the second end, the first layer having a first rib edge at the first end, and the second layer having a second rib edge at the first end, and the first layer and the second layer being interknitted along substantially the entire circumferential direction at the second end, and the second end having a third rib edge, so that the tubular part is substantially closed between the first layer and the second layer at the second end and open between the first layer and the second layer at the first end, thereby defining a room between the first layer and the second layer, the room being accessible from the first end and the room being adapted to accommodate the urine bag.

### Background art

Fixation garments for assisting in fixating urine bags to a leg of a user are known, cf. WO9633675A1, which discloses a garment for instance for fixing a urine bag to the leg of a user. The product provided by WO9633675A1 has a pocket which is adapted to completely support and fixate the urine bag during use. Even though the product has several advantages, it has turned out that it may be difficult for the wearer or caregiver to insert and position the empty urine bag in the pocket. Furthermore, due to the dual layer configuration of the product disclosed in WO9633675A1, some wearers experience the fixation garment as hot to wear.

### Summary of the invention

It is an object of the present invention to wholly or partly overcome the above disadvantages and drawbacks of the prior art. More specifically, it is an object to provide an improved fixation garment wherein positioning of the urine bag is facilitated without compromising the fixation and support of the urine bag.

The above objects, together with numerous other objects, advantages and features, which will become evident from the below description, are accomplished by a solution in accordance with the present invention by a double needle bed warp-knitted urine bag fixation garment for fixating a urine bag to a leg of a wearer, comprising
- a tubular part having a first end and a second end and a first layer and a second layer extending between the first end and the second end, the tubular part having circumferential direction, the tubular part having an upper part and a lower part, the lower part extending from the upper part towards the second end,
- the first layer having a first rib edge at the first end, and the second layer having a second rib edge at the first end,
- the first layer and the second layer being interknitted along substantially the entire circumferential direction at the second end, and the second end having a third rib edge, so that the tubular part is substantially closed between the first layer and the second layer at the second end and open between the first layer and the second layer at the first end, thereby defining a room between the first layer and the second layer, the room being accessible from the first end and the room being adapted to accommodate the urine bag,
wherein the tubular part comprises a connection in which the first layer and the second layer are connected along a part of the circumferential direction of the tubular part at a predetermined distance from the first end for providing a pocket for the urine bag in the room, the connection being adapted to maintain the urine bag in position in the pocket while it is filled during use.

Furthermore, the lower part may have a first circumference at the upper part and a second circumference at the second end, the first circumference being higher than the second circumference so that the lower part has a conical shape decreasing in circumference towards the second end. Hereby the fixation garment is provided with a better fit for the wearer, and the fixation garment has an increased fixation to the leg of the wearer, and thereby better fixation of the urine bag when it is being filled is provided. Furthermore, it is prevented that the urine bag will slide downwards due to the increasing volume as the urine bag is filled.

The connection may be provided by interknitting the first layer and the second layer.

Moreover, the connection may be provided by sewing the first layer and the second layer together.

Also, the connection may be provided by gluing the first layer and the second layer together, or by other fastening means, such as bur locks, press buttons, snap fasteners or similar devices.

Further, the connection may be extending between 10% and 70% of the circumferential direction.

In addition, the first layer and the second layer may be connected from the connection to the second end.

The connection may have an extension along the circumferential direction, the extension having a first connection end and a second connection end, the first layer and the second layer being connected from the first connection end and the second connection end, respectively, to the second end, thereby providing a part of the pocket.

Moreover, the first rib edge may have a first face and the third rib edge may have a third face, the first face and third face being adapted to be arranged opposite the wearer's leg, the first face of the first rib edge and/or the third face of the third rib edge comprising elastic yarns, such as Elastane, the elastic yarns being exposed on the first face and/or the third face in order to enhance a friction coefficient of the rib edges.

Also, an aperture may be arranged in the second layer at the second end, through which aperture a discharge pipe of the urine bag may pass.

Furthermore, the first layer may be knitted with laid-in elastic yarns, such as Elastane, for enhancing a friction coefficient of the first layer.

The first and second layers may have a content of elastic yarns of 10-40%

Additionally, the first layer and/or the second layer may comprise one or more fixation zones, the fixation zones extending in the circumferential direction of the first layer and/or the second layer.

Moreover, the fixation zones may be positioned at one or more mutual distances in a length direction of the first layer and/or the second layer.

The fixation zones arranged in a part of the tubular part extending from the connection to the second end may be arranged at a first mutual distance, and the fixation zones arranged in a part of the tubular part extending from the first end to the connection may be arranged at a second mutual distance, the first mutual distance being smaller than the second mutual distance.

Further, the fixation zones may be provided by knitting in one or more elastic yarns in the circumferential direction.

Also, the tubular part may have a length extending between the first end and the second end, the connection being arranged substantially at half the length between the first end and the second end.

In addition, a yarn having a different colour than the fixation garment may be interknitted between the third rib edge and the tubular part in approximately half of the circumferential direction for assisting the wearer in positioning the fixation garment on the leg and the urine bag in the garment. Furthermore, the coloured yarn may be used as a size indicator of the fixation garment.

The present invention also relates to a method for manufacturing a double needle bed warp-knitted urine bag fixation garment as described above, on a double needle bed knitting machine. The method comprises the steps of:
- warp-knitting an endless web,
- cutting the endless web into pieces,
- sewing a first seam extending along the upper part of the first layer, the first seam being inwardly oblique towards the second end, and
- sewing a second seam extending from the first end along the upper part of the second layer to the second end along the lower part, the second seam being straight at the upper part of the second layer and inwardly oblique from the upper part along the lower part to the second end.

In addition, the web may be after-treated with steam before it is cut into pieces.

Also, after the sewing steps, the fixation garment is ready for packaging.

### Brief description of the drawings

The invention and its many advantages will be described in more detail below with reference to the accompanying schematic drawings, which for the purpose of illustration show some non-limiting embodiments and in which
Fig. 1 shows a double needle bed warp-knitted fixation garment in a partial perspective view,
Fig. 2 shows a double needle bed warp-knitted fixation garment lying flat on a surface showing the dual layer configuration at the first end,
Fig. 3 shows the second layer folded back from the first end to the connection between the first layer and the second layer,
Fig. 4 shows the embodiment of Fig. 3 from the opposite side as shown in Fig. 3, wherein the second layer is folded back to a position wherein the urine bag may easily be inserted into the pocket, before the second layer is unfolded again, and
Figs. 5-6 show a double needle bed warp-knitted fixation garment positioned on a leg of a wearer.

All the figures are highly schematic and not necessarily to scale, and they show only those parts which are necessary in order to elucidate the invention, other parts being omitted or merely suggested.

### Detailed description of the invention

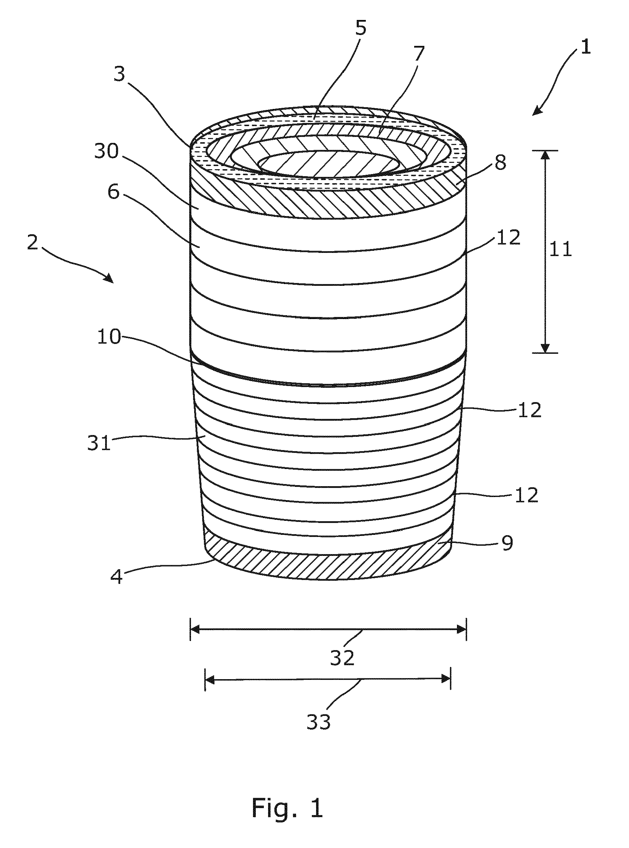

Fig. 1 shows a double needle bed warp-knitted urine bag fixation garment 1 according to the present invention. The warp-knitted fixation garment 1 is knitted on a double needle bed knitting machine. A double needle bed knitting machine is well known and will not be described any further. The fixation garment is preferably knitted in endless webs. The only kind of subsequent tailoring needed is the mutual assembling of the ends of the web sections, preferably by sewing in order to form the tubular garment. Thus the fixation garment is formed by one web section, which is folded onto itself, the ends of the web section being assembled with each other, preferably by seams in order to form a closed tubular part.

The double needle bed warp-knitted fixation garment 1 comprises a tubular part 2 having a first end 3 and a second end 4 and a first layer 5 and a second layer 6 extending between the first end 3 and the second end 4, the tubular part having circumferential direction. Furthermore, the first layer 5 has a first rib edge 7 at the first end 3, and the second layer 6 has a second rib edge 8 at the first end 3. The first layer 5 and the second layer 6 are interknitted along substantially the entire circumferential direction at the second end 4, and the second end 4 has a third rib edge 9, so that the tubular part 2 is substantially closed between the first layer 5 and the second layer 6 at the second end 4 and open between the first layer 5 and the second layer 6 at the first end, thereby defining a room between the first layer 5 and the second layer 6, the room being accessible from the first end 3 and the room being adapted to accommodate the urine bag (not shown).

According to the inventive idea, the tubular part 2 comprises a connection 10 in which the first layer 5 and the second layer 6 are connected along a part of the circumferential direction of the tubular part 2 at a predetermined distance 11 from the first end 3 for providing a pocket (not shown) for the urine bag in the room, the connection 10 being adapted to maintain the urine bag in position in the pocket while the urine bag is filled during use. Hereby it is obtained that it is easy to position the fixation garment on the wearer's leg and subsequently to insert and place a urine bag in the fixation garment 1. The connection 10 assists in defining the outer boundaries of the pocket, so that the urine bag may be maintained securely in the pocket even when it is being filled. When the urine bag is to be inserted into the fixation garment 1, the second layer 6 is folded downwards from the first end 3 towards the connection 10. The connection 10 furthermore ensures that the second layer 6 cannot be folded further down than to the connection, since the first layer 5 and the second layer 6 are connected at the connection 10. The second layer 6 may be folded downwards around the entire circumference to the connection 10. However, the first layer will remain unfolded and is fitted on the wearer's leg. When the second layer has been folded downwards, it is easier than by means of prior solutions to insert and place the urine bag between the first layer and the folded second layer, and subsequently the second layer may be unfolded again so that the urine bag will be positioned between the first layer and second layer.

The connection 10 may be provided by interknitting the first layer 5 and the second layer 6. In another embodiment, the connection 10 may be provided by sewing the first layer 5 and the second layer 6 together. Also, the connection 10 may be provided by gluing the first layer and the second layer together, or by other fastening means, such as bur locks, press buttons, snap fasteners or similar devices.

Further, the connection 10 may be extending in between 10% and 70% of the circumferential direction, depending on the size of the fixation garment. Hereby it is obtained that the urine bag may be placed along the length of the tubular part, i.e. between the first end and the second end, and the connection 10 ensures that the urine bag will not tilt sideways in the pocket, thereby preventing that the urine bag is placed along the circumferential direction of the tubular part instead of in the intended direction which is the length direction.

The tubular part 2 has an upper part 30 and a lower part 31, the lower part 31 extending from the upper part 30 towards the second end 4. The lower part 31 has a first circumference 32 at the upper part and a second circumference 33 at the second end 4, the first circumference being higher than the second circumference so that the lower part has a conical shape decreasing in circumference towards the second end. The circumferences 32, 33 are, in Fig. 1, shown as straight lines. However, they represent a circumferential extension around the tubular part. The fixation garment 1 is hereby provided with a better fit for the wearer, and the fixation garment has an increased fixation to the leg of the wearer, and thereby better fixation of the urine bag when it is being filled is provided.

Additionally, the first layer 5 and/or the second layer 6 may comprise one or more fixation zones 12, the fixation zones 12 extending in the circumferential direction of the first layer and/or the second layer. In Fig. 1, the fixation zones 12 arranged in a part of the tubular part 2 extending from the connection 10 to the second end 4 are arranged at a first mutual distance, and the fixation zones 12 arranged in a part of the tubular part 2 extending from the first end 3 to the connection 10 are arranged at a second mutual distance, the first mutual distance being smaller than the second mutual distance, so that the tubular part 2 has more fixation zones 12 than from the connection 10 to the first end 3. In the shown embodiment, the fixation zones 12 may be provided by knitting additional elastic yarns, such as Elastane, having a higher tension in the fixation zones, and/or by changing the knitting structure in the fixation zones to a more elastic or stretchable structure than the adjacent areas. In Fig. 1, the fixation zones 12 are shown as lines. However, in other embodiments, the fixation zones may have other widths in relation of the intended use of the fixation garment.

The fixation zones 12 ensure that the urine content in the urine bag will be distributed in the entire bag so that undesired outpouching of the bag is avoided. If the urine bag outpouches, it may be visible which is undesirable for the wearer of the fixation garment and the urine bag.

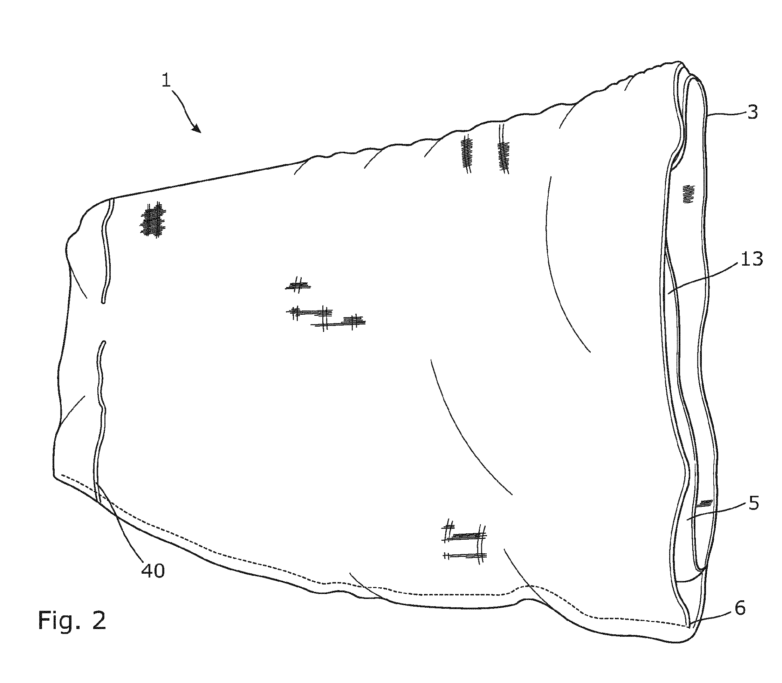

Figs. 2-5 show an embodiment of the double needle bed warp-knitted fixation garment 1 lying flat on a surface. In Fig. 2, the fixation garment 1 is shown partly from the first end 3, the first layer 5 and the second layer 6 being visible. Between the first layer 5, i.e. the inner layer, and the second layer 6, i.e. the outer layer, an opening 13 is shown.

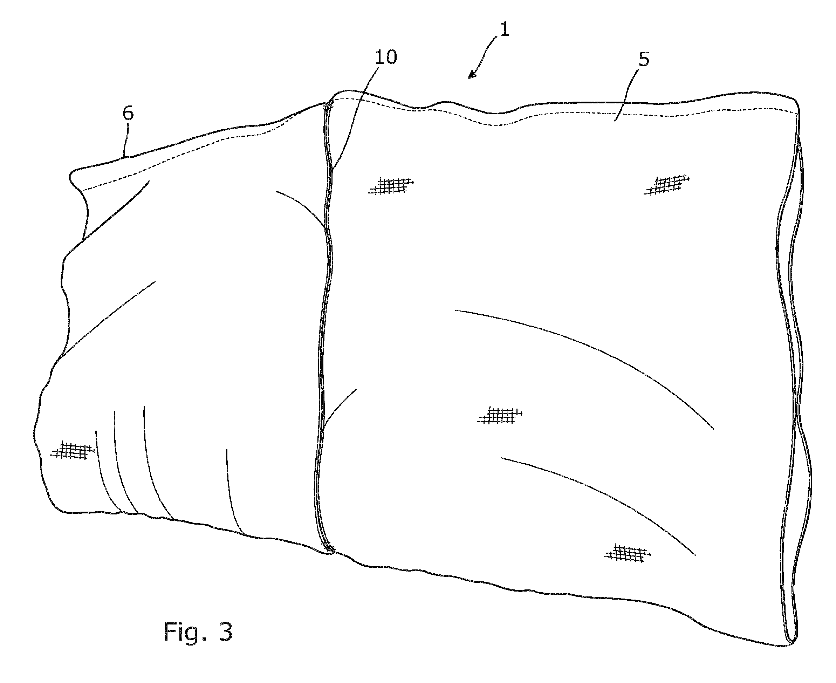

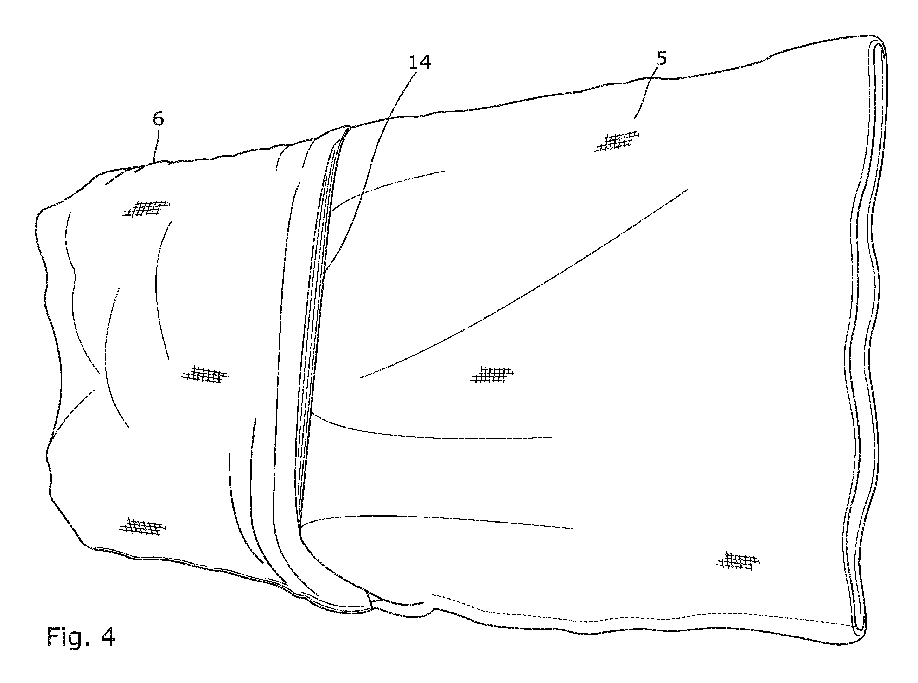

In Fig. 3, the second layer 6 is folded downwards towards the second end to the connection 10. In this embodiment, the connection 10 is extending approximately 50% of the circumference of the tubular part. Also, the tubular part may have a length extending between the first end and the second end, the connection 10 being arranged substantially at half the length between the first end and the second end. In Fig. 4, the opposite side of Fig. 3 is shown, wherein the second layer 6 is also folded downwards towards the second end 4, so that easy access to the pocket 14 is facilitated. As described above, insertion of the urine bag into the pocket 14 is facilitated since the urine bag may be pushed into the pocket 14 and the second layer 6 may then be folded back to substantially cover the urine bag. Also, an aperture (not shown) may be arranged in the second layer at the second end, through which aperture a discharge pipe of the urine bag may pass. Thus, by having the possibility of folding the second layer 6 downwards towards the second end, it is furthermore obtained that the aperture (not shown) at the second end may be accessed from the inside of the pocket 14, so that for instance the discharge pipe from the urine bag may be easily led through the aperture.

In addition, the first layer and the second layer may be connected from the connection to the second end as described briefly above in connection with the manufacturing method. The connection may have an extension along the circumferential direction, the extension having a first connection end and a second connection end, the first layer and the second layer being connected from the first connection end and the second connection end, respectively, to the second end, thereby providing a part of the pocket 14.

Moreover, the first rib edge may have a first face and the third rib edge may have a third face, the first face and third face being adapted to be arranged opposite the wearer's leg, the first face of the first rib edge and/or the third face of the third rib edge comprising elastic yarns, such as Elastane, the elastic yarns being exposed on the first face and/or the third face in order to enhance a friction coefficient of the rib edges. Hereby it is obtained that the fixation garment may be maintained in position on the wearer's leg even in circumstances where the urine bag is full.

Furthermore, the first layer may be knitted with laid-in elastic yarns, such as Elastane, for enhancing a friction coefficient of the first layer. This may be along the entire length of the first layer or in areas thereof. Furthermore, the fixation garment 1 may be knitted using different types of yarns, including for instance polyester, polypropylene, polyamide or the like, and Elastane. It will also be possible to knit in cotton or other skin-friendly material in order to improve user comfort, especially of the first layer facing the wearer's leg.

In addition, a yarn or area 40 (as shown in Figs. 2, 5 and 6) having a different colour than the fixation garment may be interknitted between the third rib edge and the tubular part in approximately half of the circumferential direction for assisting the wearer in positioning the fixation garment on the leg and the urine bag in the fixation garment. Different colours of the coloured yarns may indicate different sizes of the fixation garment, so that it is easy for the wearer or caregiver to determine the size of the fixation garment.

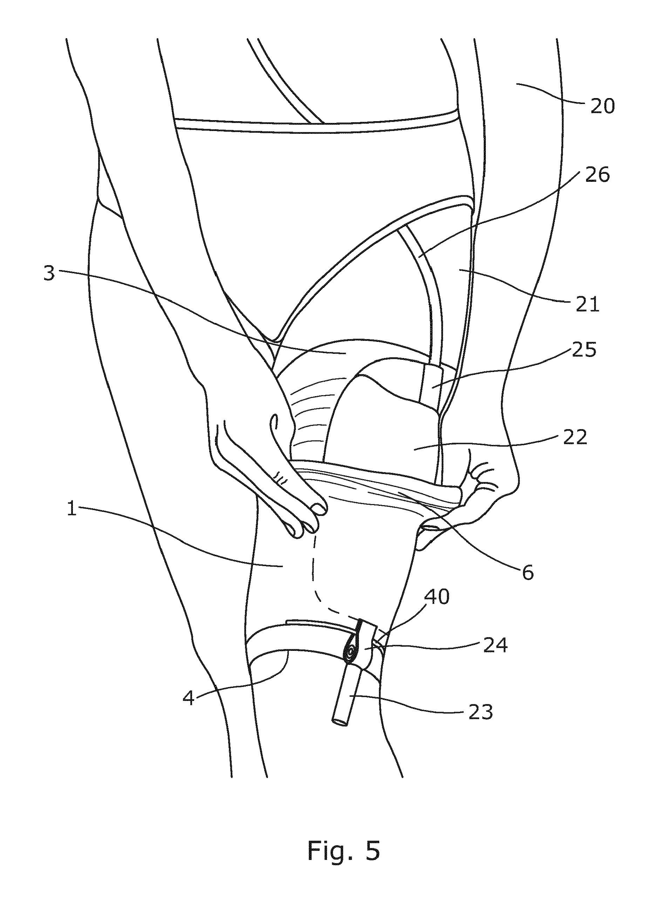

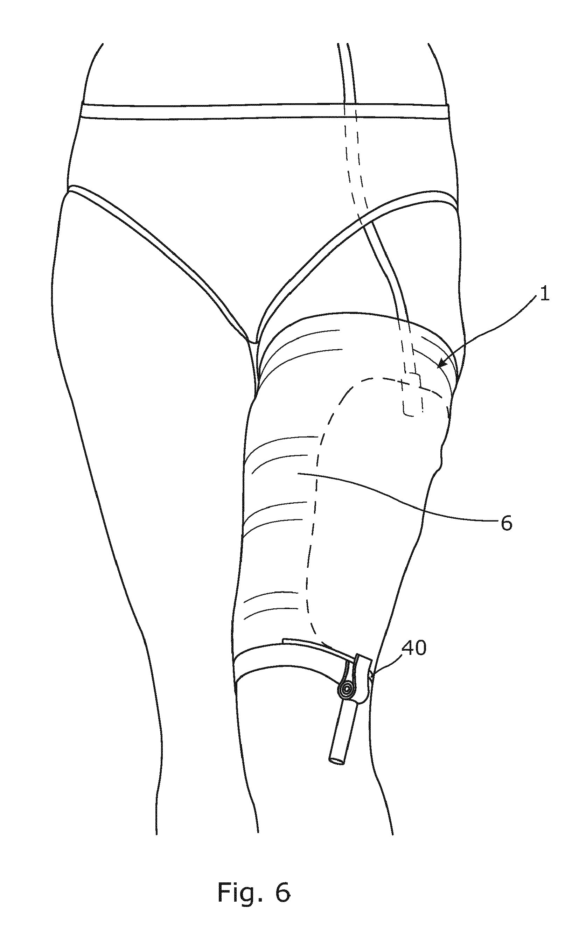

Figs. 5 and 6 show how the double needle bed warp-knitted fixation garment 1 is used. In Fig. 5, the fixation garment 1 has been pulled up and positioned on a wearer's 20 leg 21. The urine bag 22 has been inserted into the pocket, and the discharge pipe 23 is extending out of the aperture 24 at the second end 4. The second layer 6 has been folded downwards as described above for facilitating the insertion og positioning of the urine bag 22 and is, in Fig. 5, about to be unfolded by the wearer 20. The urine bag 22 has an inlet 25 connected with a tube 26, which is extending out of the pocket at the first end 3.

In Fig. 6, the second layer 6 has been unfolded and is now covering the urine bag, which then is properly fixated and positioned by the fixation garment 1 according to the present invention.

The above-mentioned fixation garment 1 may be manufactured by the following method on a double needle bed knitting machine. The method comprises the steps of:
- warp-knitting an endless web,
- cutting the endless web into pieces,
- sewing a first seam extending along the upper part of the first layer, the first seam being inwardly oblique towards the second end, and
- sewing a second seam extending from the first end along the upper part of the second layer to the second end along the lower part, the second seam being straight at the upper part of the second layer and inwardly oblique from the upper part along the lower part to the second end.

The web may be after-treated with steam before it is cut into pieces.

After the sewing steps, the fixation garment is ready for packaging.

Although the invention has been described in the above in connection with preferred embodiments of the invention, it will be evident for a person skilled in the art that several modifications are conceivable without departing from the invention as defined by the following claims.

## Claims

1. A double needle bed warp-knitted urine bag fixation garment (1) for fixating a urine bag (22) to a leg (21) of a wearer (20), comprising
- a tubular part (2) having a first end (3) and a second end (4) and a first layer (5) and a second layer (6) extending between the first end (3) and the second end (4), the tubular part (2) having circumferential direction, the tubular part having an upper part and a lower part, the lower part extending from the upper part towards the second end,
- the first layer (5) having a first rib edge (7) at the first end, and the second layer having a second rib edge (8) at the first end,
- the first layer and the second layer being interknitted along substantially the entire circumferential direction at the second end, and the second end having a third rib edge (9), so that the tubular part is substantially closed between the first layer and the second layer at the second end and open between the first layer and the second layer at the first end, thereby defining a room between the first layer and the second layer, the room being accessible from the first end and the room being adapted to accommodate the urine bag,
wherein the tubular part (2) comprises a connection (10) in which the first layer and the second layer are connected along a part of the circumferential direction of the tubular part at a predetermined distance from the first end for providing a pocket (14) for the urine bag in the room, the connection being adapted to maintain the urine bag in position in the pocket while it is filled during use.

2. A double needle bed warp-knitted urine bag fixation garment (1) according to claim 1, wherein the lower part has a first circumference at the upper part and a second circumference at the second end, the first circumference being higher than the second circumference so that the lower part has a conical shape decreasing in circumference towards the second end.

3. A double needle bed warp-knitted urine bag fixation garment according to claim 1 or 2, wherein the connection (10) is provided by interknitting the first layer and the second layer.

4. A double needle bed warp-knitted urine bag fixation garment according to any of the preceding claims, wherein the connection (10) is extending in between 10% and 70% of the circumferential direction.

5. A double needle bed warp-knitted urine bag fixation garment according to any of the preceding claims, wherein the first layer and the second layer are connected from the connection to the second end.

6. A double needle bed warp-knitted urine bag fixation garment according to any of the preceding claims, wherein the connection has an extension along the circumferential direction, the extension having a first connection end and a second connection end, the first layer and the second layer being connected from the first connection end and the second connection end, respectively, to the second end, thereby providing a part of the pocket.

7. A double needle bed warp-knitted urine bag fixation garment according any of the preceding claims, wherein the first rib edge has a first face and the third rib edge has a third face, the first face and third face being adapted to be arranged opposite the wearer's leg, the first face of the first rib edge and/or the third face of the third rib edge comprising elastic yarns, such as Elastane, the elastic yarns being exposed on the first face and/or the third face in order to enhance a friction coefficient of the rib edges.

8. A double needle bed warp-knitted urine bag fixation garment according to any of the preceding claims, wherein an aperture is arranged in the second layer at the second end, through which aperture a discharge pipe of the urine bag may pass.

9. A double needle bed warp-knitted urine bag fixation garment according to any of the preceding claims, wherein the first layer is knitted with laid-in elastic yarns, such as Elastane, for enhancing a friction coefficient of the first layer.

10. A double needle bed warp-knitted urine bag fixation garment according to any of the preceding claims, wherein the first layer and/or the second layer comprise one or more fixation zones (12), the fixation zones extending in the circumferential direction of the first layer and/or the second layer.

11. A double needle bed warp-knitted urine bag fixation garment according to claim 10, wherein the fixation zones (12) are positioned at one or more mutual distances in a length direction of the first layer and/or the second layer.

12. A double needle bed warp-knitted urine bag fixation garment according to claim 10 or 11, wherein the fixation zones arranged in a part of the tubular part extending from the connection to the second end are arranged at a first mutual distance, and the fixation zones arranged in a part of the tubular part extending from the first end to the connection are arranged at a second mutual distance, the first mutual distance being smaller than the second mutual distance.

13. A double needle bed warp-knitted urine bag fixation garment according to any of the claims 10-12, wherein the fixation zones are provided by knitting in one or more elastic yarns in the circumferential direction.

14. A double needle bed warp-knitted urine bag fixation garment according to any of the preceding claims, wherein a yarn having a different colour than the fixation garment is interknitted between the third rib edge and the tubular part in approximately half of the circumferential direction for assisting the wearer in positioning the fixation garment on the leg and the urine bag in the garment.

15. A method for manufacturing a double needle bed warp-knitted urine bag fixation garment according to any of the preceding claims on a double needle bed knitting machine, comprising the steps of:
- warp-knitting an endless web,
- cutting the endless web into pieces,
- sewing a first seam extending along the upper part of the first layer, the first seam being inwardly oblique towards the second end, and
- sewing a second seam extending from the first end along the upper part of the second layer to the second end along the lower part, the second seam being straight at the upper part of the second layer and inwardly oblique from the upper part along the lower part to the second end.
